# EUROPEAN PATENT APPLICATION

(11) **EP 2 008 663 A1**
(43) Date of publication of application: **31.12.2008**
(21) Application number: 07739326.2
(22) Date of filing: 22.03.2007
(51) Int. Cl.: A61K 38/00, A61K 9/08, A61K 9/70, A61K 31/734, A61P 17/02

(54) **LIQUID COMPOSITION FOR EXTERNAL APPLICATION AND PREPARATION FOR EXTERNAL APPLICATION FOR TREATMENT OF SKIN ULCER**

(30) Priority: 22.03.2006 JP 2006079614
(71) Applicant: Cellgentech, Inc., Chiyoda-ku Tokyo 101-0052 (JP)
(72) Inventor: JIMI, Shiro, Fukuoka-shi, Fukuoka 814-0175 (JP); SUZUMIYA, Junji, Fukuoka-shi, Fukuoka 814-0155 (JP)
(74) Representative: Walcher, Armin
(86) International application number: PCT/JP2007/055883
(87) International publication number: WO 2007/119486

(57) **Abstract**

An object of the present invention is to provide a liquid composition for external application and a preparation for external application which are excellent in healing effect on skin ulcers including intractable skin ulcers such as bedsore (decubitus), diabetic skin ulcer and ischemic skin ulcer. The liquid composition for external application of the present invention as means for the resolution is **characterized by** comprising at least G-CSF (a granulocyte colony stimulating factor) as an active ingredient and 0.001% to 5% of alginic. acid. The preparation for external application for treatment of a skin ulcer of the present invention is **characterized by** comprising the liquid composition for external application of the present invention as a medicinal ingredient.

## Description

### Technical Field

The present invention relates to a liquid composition for external application and a preparation for external application which are excellent in healing effect on skin ulcers including intractable skin ulcers such as bedsore (decubitus), diabetic skin ulcer and ischemic skin ulcer.

### Background Art

At present, in advanced countries including Japan, lifestyle-related diseases including diabetes are progressively increasing due to aging of society and changes in lifestyles and the like. When hospitalized patients or bedridden patients suffer from circulatory disorder due to diabetes or arteriosclerosis, bedsore is formed. Because bedsore is intractable, there has not been an excellent therapeutic method so far, therefore, the development of an effective therapeutic method has been awaited. Bedsore starts to occur at the sacral region or heel region, and the causes of the occurrence of bedsore are supposed to be a decrease in tissue endurance due to compression, wetting, malnutrition and the like. In particular, skin tissue and subcutaneous tissue become thin and vulnerable in elderly, and a decrease in the tissue healing response is to be a cause of further delaying the bedsore healing. The causes of intractable skin ulcers including bedsore have been believed to be a decrease in angiogenesis potential, a disorder of proliferation of fibroblasts, etc., that is, a decrease in the ability to form granulation tissue in an affected area. However, the exact mechanism of granulation tissue formation has not been elucidated yet. Therefore, the current situation is that only a method for maintaining tissue in an affected area, direct spraying of growth factors or the like has been employed as the therapeutic method.

Recently, it has been reported that a wound healing effect can be obtained by intraperitoneal injection of G-CSF (a granulocyte colony stimulating factor) (Non-patent document 1). Further, in Patent document 1, a method of accelerating wound healing by local application or parenteral administration of GM-CSF (a granulocyte macrophage colony stimulating factor) or G-CSF has been proposed. However, either of the documents does not study the healing effect on intractable skin ulcers. Moreover, Patent document 1 does not describe data showing a wound healing effect of an agent for external application containing G-CSF as an active ingredient in the first place. Further, according to the study by the present inventors, it has been revealed that GM-CSF for which data showing a wound healing effect by local application to the surface area of a cut is described in Patent document 1 does not have an effect on intractable skin ulcers.
Patent document 1: JP-T-5-506673
Non-patent document 1: Eroglu E, et al., Effects of granulocyte-colony stimulating factor on wound healing in a mouse model of burn trauma. Tohoku J. Med. 204, 11-16 (2004)

### Disclosure of the invention

### Problems that the Invention is to Solve

Accordingly, an object of the present invention is to provide a liquid composition for external application and a preparation for external application which are excellent in healing effect on skin ulcers including intractable skin ulcers such as bedsore (decubitus), diabetic skin ulcer and ischemic skin ulcer.

### Means for Solving the Problems

The present inventors conducted intensive studies in view of the above points, and as a result, they found that a solution of alginic acid at a low concentration itself exhibits an action of increasing the amount of blood flow in an affected area by applying the solution to a surface area of an intractable skin ulcer, and also found that by applying a mixture of the solution with G-CSF, the amount of G-CSF present in the blood is increased or the decrement of G-CSF in the blood is suppressed for a given period of time, the amount of blood flow in an affected area is increased, or the like, resulting in contribution to improvement of the healing effect on an intractable skin ulcer, and that even if the solution is mixed with G-CSF, white turbidity is not caused and a liquid composition with high transparency can be formed. Alginic acid is a substance which has already been used as a moisturizing and protecting ingredient or the like in cosmetics and drugs. However, there has been no report on the above-mentioned properties of the solution of alginic acid at a low concentration, which were found for the first time by the present inventors.

A liquid composition for external application of the present invention which has been made based on the above-mentioned findings is characterized in that, as described in claim 1, it comprises at least G-CSF as an active ingredient and 0.001% to 5% of alginic acid.
Further, the liquid composition for external application as described in claim 2 is characterized in that, in the liquid composition for external application described in claim 1, the concentration of G-CSF is 10 µg/mL to 2 mg/mL.
Further, the liquid composition for external application as described in claim 3 is characterized in that, in the liquid composition for external application described in claim 1, the concentration of alginic acid is 0.01% to 3%.
Further, the liquid composition for external application as described in claim 4 is characterized in that, in the liquid composition for external application described in claim 1, the turbidity is 0.04 to 0.06.
Further, a preparation for external application for treatment of a skin ulcer of the present invention is characterized in that, as described in claim 5, it comprises the liquid composition for external application described in any one of claims 1 to 4 as a medicinal ingredient.
Further, the preparation for external application as described in claim 6 is characterized in that, in the preparation for external application described in claim 5, the formulation of the preparation is a lotion.
Further, the preparation for external application as described in claim 7 is characterized in that, in the preparation for external application described in claim 5, the formulation of the preparation is a sheet-formed preparation.

### Effect of the Invention

According to the present invention, a liquid composition for external application and a preparation for external application which are excellent in healing effect on skin ulcers including intractable skin ulcers such as bedsore (decubitus), diabetic skin ulcer and ischemic skin ulcer are provided.

### Brief Description of the Drawings

[Fig. 1] A graph showing a change over time of the concentration of rhG-CSF in the peripheral blood by applying a liquid composition containing rhG-CSF and alginic acid at different concentrations to a surface area of an intractable skin ulcer in Example.
[Fig. 2] A graph showing a relationship between the concentration of alginic acid and turbidity in Example.

### Best Mode for Carrying Out the Invention

G-CSF to be an active ingredient of the liquid composition for external application of the present invention is not limited to a human-derived natural product having a known amino acid sequence, and may be a product produced by a genetic engineering technique, an analogue in which one or more amino acids have been substituted, deleted, added or inserted in the amino acid sequence of the natural product, or a chemically modified product thereof, as long as it can be recognized as G-CSF by a person skilled in the art, for example, it has an action of differentiating and proliferating granulocytes. The main action of G-CSF in the wound healing is to help mobilization of the whole tissue repair cells such as neutrophils, fibrocytes and megakaryocytes from the bone marrow. This action can be expected to be reinforced by using another vascular endothelial cell growth factor, for example, a fibroblast growth factor (FGF) or the like in combination, and can further amplify the ability to form granulation tissue. Therefore, the administration of G-CSF increases a plurality of bone marrow-derived cells required for tissue repair in the blood for an intractable skin ulcer in which the ability to heal wound has been lowered. As a result, the maximum potential healing ability in a wound area is exhibited, whereby a basic response for healing an intractable skin ulcer is accelerated. Further, as an effect of local application of G-CSF to an area of a skin ulcer through application, spraying or the like, a wound healing accelerating effect by acceleration of differentiation of bone marrow-derived cells with low differentiation such as stem cells infiltrated into the vicinity of the area of a skin ulcer is also possible. The concentration of G-CSF contained in the liquid composition for external application is preferably 10 µg/mL to 2 mg/mL, more preferably 25 µg/mL to 1 mg/mL, and further more preferably 50 µg/mL to 500 µg/mL. When the concentration is too low, the action of G-CSF is not exhibited. On the other hand, when the concentration is too high, the action is not improved so much.

The liquid composition for external application of the present invention is characterized by containing alginic acid at a low concentration, and the concentration of alginic acid is 0.001% to 5% (% by weight concentration). The concentration is preferably 0.01% to 3%, more preferably 0.05% to 1%, and further more preferably 0.1% to 0.5%. Surprisingly, if the concentration of alginic acid is too low or too high, it does not exhibit an action of increasing the amount of blood flow in an affected area when it is applied to a surface area of a skin ulcer. Further, when the concentration is too high, white turbidity is caused and a liquid composition with high transparency (for example, the turbidity is 0.04 to 0.06) cannot be formed, and moreover, the viscosity is increased and a problem related to handling arises such that spraying becomes difficult. On the other hand, when the concentration is too low, the viscosity is decreased and a problem arises such that G-CSF cannot be sufficiently retained on a surface area of a skin ulcer (accordingly, the viscosity is preferably about 1.5 5 mPa·s to 20 mPa·s).

The liquid composition for external application of the present invention may be prepared by, for example, dissolving G-CSF and alginic acid or a salt thereof (such as a sodium salt, a potassium salt or an ammonium salt) in a solvent such as purified water, a saline solution, a phosphate buffer or a hydrochloric acid solution at the above-mentioned concentration. Incidentally, a surfactant such as polysorbate, a stabilizer such as mannitol or the like may be added as an additive if necessary. The pH of the liquid composition for external application of the present invention is preferably 2.5 to 5.0 for stably keeping G-CSF in the composition. The adjustment of the pH may be performed by using an inorganic acid such as hydrochloric acid or an organic acid such as citric acid if necessary. The liquid composition for external application of the present invention can be used by being formulated into a preparation such as a liquid, a lotion or a propellant (a spray) according to a commonly used procedure. It goes without saying that various components such as a well-known stabilizer, thickener, solubilizer, preservative, filler, tonicity agent, disinfectant, antiseptic and gelling agent can be added upon formulation of preparations. The liquid composition for external application of the present invention can be expected to exhibit an excellent healing effect by applying or spraying this composition to a surface area of an intractable skin ulcer, for example, once to several times a day or once per one day to seven days for one week to one month at a dose (in terms of G-CSF) of preferably 0.1 µg/cm² to 500 µg/cm², more preferably 0.5 µg/cm² to 100 µg/cm², and further more preferably 1 µg/cm² to 10 µg/cm². Further, the liquid composition for external application of the present invention may be formulated into a sheet-formed preparation by impregnating it into a base material sheet such as a collagen sheet (obtained by processing collagen by spinning into a form like a cotton fiber) or an alginate sheet (obtained by processing alginate into a fiber and forming a non-woven cloth). By using the liquid composition in this way, an excellent healing effect on an intractable skin ulcer can be expected in combination with the moisturizing property, the wound healing accelerating effect and the like of the base material sheet (the dose of G-CSF to be applied to a wound area is the same as above).

### Examples

Hereinafter, the present invention will be described in detail with reference to Examples. However, it shall be noted that the present invention should not be construed to be limited to the following description.

### Example 1: Evaluation of healing effect of liquid composition on intractable skin ulcer (1)

### 1: Preparation of liquid composition

The following liquid compositions were prepared by using a recombinant human G-CSF preparation (trade name: Gran Injection, hereinafter referred to as "rhG-CSF") manufactured by Kirin Brewery. Co., Ltd., sodium alginate and a saline solution. The pH and viscosity of each liquid composition are shown in Table 1.
(a) a liquid composition in which the concentration of rhG-CSF is 100 µg/mL and alginic acid is not contained
(b) a liquid composition in which the concentration of rhG-CSF is 100 µg/mL and the concentration of alginic acid is 0.0625%
(c) a liquid composition in which the concentration of rhG-CSF is 100 µg/mL and the concentration of alginic acid is 0.125%
(d) a liquid composition in which the concentration of rhG-CSF is 100 µg/mL and the concentration of alginic acid is 0.25%
(e) a liquid composition in which the concentration of rhG-CSF is 100 µg/mL and the concentration of alginic acid is 0.5%
(f) a liquid composition in which the concentration of rhG-CSF is 100 µg/mL and the concentration of alginic acid is 1.0%

**[Table 1]**

| Liquid composition | pH | Viscosity (mPa·s) |
|---|---|---|
| (a) | 4.04 | 1.13 |
| (b) | 4.59 | 1.96 |
| (c) | 4.66 | 2.42 |
| (d) | 4.75 | 3.11 |
| (e) | 4.86 | 5.32 |
| (f) | 4.99 | 12.3 |

### 2: Production of intractable skin ulcer model

By using a C57BL/6 mouse, an ulcer model of delayed skin wound healing as an intractable skin ulcer model was produced. Specifically, full thickness dorsal skin tissue with a size of 2 × 1.5 cm of a C57BL/6 mouse was excised, whereby the fascia was exposed with almost no bleeding. Then, the wound area was completely covered with cotton which had been sufficiently soaked in 70% ethanol, whereby ethanol exposure for 30 sec, 60 sec, 300 sec and 600 sec was carried out. After that, the wound area was air-dried, and the area of the wound area was measured (day 0). 7 days after completion of the experiment, the area of the wound area was measured, which was compared with the area of the wound area on day 0, and a change in the area of the wound area was examined. As a result, the shrinkage of the wound area was reduced as the time of ethanol exposure was increased, and the shrinkage hardly occurred by the 300 sec exposure. Incidentally, the mouse subjected to 600 sec ethanol exposure died at 3 days after the experiment due to deterioration of systemic symptoms. In the case where the time of ethanol exposure was short such as 30 sec exposure or 60 sec exposure, the healing was delayed because the tissue around the wound area directly received degenerative damage due to ethanol, however, granulation tissue was formed in the wound area in one week after the ethanol exposure, and the wound area was covered with epidermal tissue in about 3 weeks and the healing was completed. The 60 sec exposure was to be a factor of delaying the shrinkage of the wound area, but there was no systemic symptom and its effect was sufficient, therefore, this mouse was adopted as an ulcer model of delayed skin wound healing.

### 3: Evaluation of change over time of concentration of rhG-CSF in peripheral blood by application to surface area of intractable skin ulcer

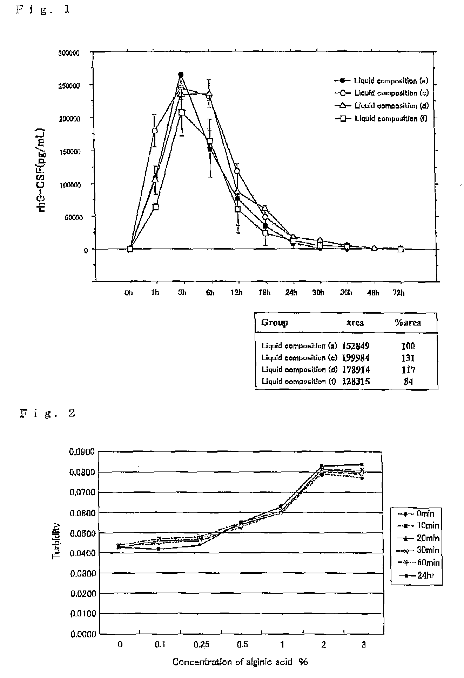

To the affected area of the above-mentioned ulcer model of delayed skin wound healing (60 sec ethanol exposure model), 100 µL of any of 4 types of liquid compositions: liquid composition (a), liquid composition (c), liquid composition (d), and liquid composition (f) was applied (in each group, n = 2). The blood was collected from the orbit of the mouse under ether anesthesia at every lapse of a predetermined time period, and the plasma was recovered and stored. A calibration curve was created by the used rhG-CSF, and the concentration in the plasma was measured by the ELISA method for human G-CSF which shows no cross-reactivity with mouse G-CSF. The results are shown in Fig. 1 as a graph. As is apparent from Fig. 1, rhG-CSF appeared in the peripheral blood from 1 hour after application in all groups with application of the liquid compositions, and the peak was observed after 3 hours, then, rhG-CSF almost disappeared at 72 hours. The area representing the amount of rhG-CSF present in the peripheral blood in each group with application of the liquid compositions was measured from the graph. Then, the area of the group with application of liquid composition (a) was taken to be 100% and the area of the other groups with application of the liquid compositions was calculated. As a result, the area of the group with application of liquid composition (c) containing alginic acid at the lowest concentration was 131%, which was the largest, the area of the group with application of liquid composition (d) was 117%, and the area of the group with application of liquid composition (f) was 84%, which was smaller than that of the group with application of liquid composition (a). The decrement of rhG-CSF in the blood over time was suppressed in the group with application of liquid composition (c) and the group with application of liquid composition (d) compared with the group with application of liquid composition (a). From the above results, it was found that alginic acid at a low concentration has an effect on increasing the amount of G-CSF present in the blood or suppressing the decrement of G-CSF in the blood for a given period of time.

### 4: Evaluation of action of increasing amount of blood flow in affected area by application to surface area of intractable skin ulcer

To the affected area of the above-mentioned ulcer model of delayed skin wound healing (60 sec ethanol exposure model), 100 µL of any of 4 types of liquid compositions: liquid composition (a), liquid composition (b), liquid composition (c), and liquid composition (e) was applied (in each group, n = 2). After 3 days, the blood flow in the affected area was measured under ether anesthesia by using Laser Doppler Blood Flow Imager (Monte System Corporation: Moor LD 12-IR). As a result, in the group with application of liquid composition (a), a slight increase in the blood flow was observed compared with the group with application of a saline solution as a control group. In the groups with application of liquid composition (b), liquid composition (c) and liquid composition (e), an apparent increase in the blood flow was observed in the entire affected area, and particularly in the groups with application of liquid composition (b) and liquid composition (c), the effect was evident.

### 5: Evaluation of healing effect of application to surface area of intractable skin ulcer

To the affected area of the above-mentioned ulcer model of delayed skin wound healing (60 sec ethanol exposure model), 100 µL of either of 2 types of liquid compositions: liquid composition (a) and liquid composition (c) was applied, and the healing effects were compared. As a result, the ability to form granulation tissue and neovessels was increased and the healing rate was superior in the group with application of liquid composition (c).

### Example 2: Evaluation of healing effect of liquid composition on intractable skin ulcer (2)

By using a C57BL/6 mouse, an intractable diabetic skin ulcer model as an intractable skin ulcer model was produced. Specifically, a diabetes model with hyperglycemia was produced by intraperitoneally administering streptozotocin (STZ) to a C57BL/6 mouse at a dose of 4 mg/20 g of body weight using a 0.2 M citrate buffer (pH 4.8) as a solvent. By the STZ administration, bone marrow formation was temporarily suppressed, however, it was recovered after 2 weeks. 3 weeks after STZ administration, full thickness dorsal skin tissue with a size of 2 × 1.5 cm of the mouse was excised and exposure of 70% ethanol was carried out for 60 sec, whereby an intractable diabetic skin ulcer model was produced. 150 µL of an atelocollagen solution (a 3 mg/mL hydrochloric acid solution) was applied to a wound area of this model and the solution was coagulated. Then, 200 µL of a liquid composition, which was prepared by using a recombinant human G-CSF preparation (trade name: Neutrogin Injection, hereinafter referred to as "rhG-CSF") manufactured by Chugai Pharmaceutical Co. , Ltd., sodium alginate and a saline solution and in which the concentration of rhG-CSF was 50 µg/mL and the concentration of alginic acid was 1%, was applied to the affected area, and an effect on the shrinkage of the wound area was studied. As a result, this liquid composition shrank the wound area more effectively than in the case where a saline solution was applied as a control.

### Example 3: Evaluation of transparency of liquid composition

By using a recombinant human G-CSF preparation (trade name: Gran Injection, hereinafter referred to as "rhG-CSF") manufactured by Kirin Brewery. Co., Ltd., sodium alginate and purified water, liquid compositions containing 85 µg/mL of rhG-CSF and alginic acid at various concentrations were prepared, and the turbidity of the liquid compositions was measured at every lapse of a predetermined time period by using Absorbance Microplate Reader (Tecan Inc.: Sunrise Remote). The results are shown in Fig. 2 as a graph. As is apparent from Fig. 2, it was found that if the concentration of alginic acid is lower than 1%, the resulting liquid composition has a turbidity of 0.06 or less and has high transparency. Accordingly, from the above-mentioned Example 1 and Example 2, and this Example 3, it was found that the range of the concentration of alginic acid at which a pharmacological effect of incorporation of alginic acid in a liquid composition containing rhG-CSF as an active ingredient is exhibited and the range of the concentration of alginic acid at which high transparency of the liquid composition can be maintained conveniently coincide with each other.

### Reference example 1: Evaluation of action of increasing amount of blood flow in affected area by application of solution of alginic acid at low concentration to surface area of intractable skin ulcer

By using sodium alginate and a saline solution, solutions of alginic acid in a concentration of 0%, 0.125%, 0.25%, 0.5% and 1% were prepared, and evaluation was performed in the same manner as in Example 1-4. As a result, it was found that the solutions of alginic acid in a concentration of 0.125%, 0.25% and 0.5% have a significant action of increasing the amount of blood flow in the affected area.

### Reference example 2: Evaluation of healing effect of solution of alginic acid at low concentration on intractable diabetic skin ulcer model

An effect of a liquid composition containing 1% of alginic acid prepared by using, sodium alginate and a saline solution on the shrinkage of a wound area was studied in the same manner as in Example 2. As a result, the effect of the liquid composition on the shrinkage of the wound area was superior to in the case where a saline solution was applied as a control, and therefore it was found that a solution of alginic acid at a low concentration itself has an effect on the shrinkage of a wound area.

### Reference example 3: Evaluation of cytotoxic action of alginic acid to cultured cells

Alginic acid was added to culture solutions (RPMI 1640 + 10% FBS) of cultured histiocytes (U937) and cultured promyelocytes (HL60), and the culture solutions were incubated for 3 days. Then, the ratio of viable cells was determined by using GUAVA ViaCount based on flow cytometry. As a result, cytotoxicity to both cells was not observed in the culture medium containing up to 0.5% of alginic acid.

### Reference example 4: Evaluation of healing effect of GM-CSF external agent on intractable skin ulcer

As GM-CSF, a mouse-derived recombinant (R&D System) was used. A solution obtained by dissolving this GM-CSF in an atelocollagen solution at a concentration of 20 µg/100 µL was applied to an affected area of the above-mentioned ulcer model of delayed skin wound healing (60 sec ethanol exposure model) and coagulated, and evaluation was performed in the healing process for 7 days. Further, a group in which only an atelocollagen solution (a 3 mg/mL hydrochloric acid solution) was applied to the affected area and coagulated was used as a control. As a result, throughout the 7-day period of the study, the wound area had symptoms like edema and so-called "swelling" was noted in the GM-CSF administration group compared with the control group. When the shrinkage of the wound area after 7 days was examined, the relative area of the wound area in the GM-CSF administration group was larger by about 15% than that of the control group, and the shrinking action of natural healing of a wound area was inhibited, and the wound area was exacerbated. When the thickness of granulation tissue was examined, the thickness thereof in the GM-CSF administration group was slightly thicker than that of the control group. However, histologically, while proliferation of blood vessels, infiltration of inflammatory cells and proliferation of spindle-shaped fibroblasts were notably observed in the granulation tissue in the control group, edema was noted in the GM-CSF administration group. From the above results, it was found that in the ulcer model of delayed skin wound healing, the GM-CSF external agent does not have a wound healing accelerating effect and rather enhances edema due to inflammation, and has an adverse effect on wound healing.

### Preparation example 1: Lotion (1)

| | (g/100 mL) |
|---|---|
| Glycerin | 10 |
| Ethanol | 10 |
| 1,3-butylene glycol | 5 |
| Hydroxyethyl cellulose | 1 |
| Cetanol | 1 |
| G-CSF | 0.02 |
| Atelocollagen | 0.3 |
| Sodium alginate | 0.25 |
| Citric acid monohydrate | 0.66 |
| Trisodium citrate dihydrate | 0.27 |
| Methyl parahydroxybenzoate | 0.1 |
| Sodium dihydrogen phosphate | 3 |
| Purified water | 68.4 |

A lotion for treatment of a skin ulcer that has the above-mentioned composition was prepared by a well-known manufacturing process for a lotion.

### Preparation example 2: Lotion (2)

| | (g/100 mL) |
|---|---|
| Glycerin | 10 |
| Ethanol | 10 |
| 1,3-butylene glycol | 5 |
| Hydroxyethyl cellulose | 1 |
| Cetanol | 1 |
| G-CSF | 0.02 |
| Sodium alginate | 0.25 |
| Citric acid monohydrate | 0.66 |
| Trisodium citrate dihydrate | 0.27 |
| Methyl parahydroxybenzoate | 0.1 |
| Sodium dihydrogen phosphate | 3 |
| Purified water | 68.7 |

A lotion for treatment of a skin ulcer that has the above-mentioned composition was prepared by a well-known manufacturing process for a lotion.

### Preparation example 3: Sheet-formed preparation (1)

| | (g/100 mL) |
|---|---|
| Glycerin | 7 |
| 1,3-butylene glycol | 3 |
| Pentylene glycol | 5 |
| Phenoxyethanol | 0.5 |
| Methylparaben | 0.1 |
| G-CSF | 0.02 |
| Sodium alginate | 0.5 |
| Sodium dihydrogen phosphate | 3 |
| Purified water | 80.88 |

A sheet-formed preparation for treatment of a skin ulcer was prepared by impregnating a medicinal concentrated solution that has the above-mentioned composition into a collagen sheet with low antigenicity (Nippon Zoki Pharmaceutical Co., Ltd.: Integran) in which atelocollagen (collagen obtained by removing the antigenicity expression site with a protease) was processed by spinning into a form like a cotton fiber.

### Preparation example 4: Sheet-formed preparation (2)

A sheet-formed preparation for treatment of a skin ulcer was prepared in the same manner as in Preparation example 3 except that an alginate sheet (Kaltostat manufactured by convatec Inc. in which a mixed salt of calcium alginate and sodium alginate was processed into a fiber and formed a non-woven cloth) was used in place of the collagen sheet in Preparation example 3.

### Industrial Applicability

The present invention has industrial applicability in the point that it can provide a liquid composition for external application and a preparation for external application which are excellent in healing effect on skin ulcers including intractable skin ulcers such as bedsore (decubitus), diabetic skin ulcer and ischemic skin ulcer.

## Claims

1. A liquid composition for external application, comprising at least G-CSF (a granulocyte colony stimulating factor) as an active ingredient and 0.001% to 5% of alginic acid.

2. The liquid composition for external application according to claim 1, wherein the concentration of G-CSF is 10 µg/mL to 2 mg/mL.

3. The liquid composition for external application according to claim 1, wherein the concentration of alginic acid is 0.01% to 3%.

4. The liquid composition for external application according to claim 1, wherein the turbidity is 0.04 to 0.06.

5. A preparation for external application for treatment of a skin ulcer, comprising the liquid composition for external application according to any one of claims 1 to 4 as a medicinal ingredient.

6. The preparation for external application according to claim 5, wherein the formulation of the preparation is a lotion.

7. The preparation for external application according to claim 5, wherein the formulation of the preparation is a sheet-formed preparation.
